# FASCICULE DE BREVET EUROPEEN

(11) **EP 1 150 649 B1**
(45) Date de publication et mention de la délivrance du brevet: **24.11.2004**
(21) Numéro de dépôt: 00901711.2
(22) Date de dépôt: 03.02.2000
(51) Int. Cl.: A61K 7/42

(54) **COMPOSITIONS COSMETIQUES POUR LA PHOTOPROTECTION DE LA PEAU ET/OU DES CHEVEUX CONTENANT UN DERIVE SILICONE DE BENZOTRIAZOLE ET UNE BIS-RESORCINYL TRIAZINE**
KOSMETISCHE MITTEL ZUM LICHTSCHUTZ DER HAUT UND DER HAARE, DIE EIN BENZOTRIAZOL-SILIKONDERIVAT UND EIN BISRESORCINYLTRIAZINDERIVAT ENTHALTEN
COSMETIC COMPOSITIONS FOR PHOTOPROTECTION OF THE SKIN AND/OR HAIR CONTAINING A BENZOTRIAZOLE SILICONE DERIVATIVE AND A TRIAZINE BIS-RESORCINYL DERIVATIVE

(30) Priorité: 12.02.1999 FR 9901729
(43) Date de publication de la demande: 07.11.2001
(73) Titulaire: L'OREAL, 75008 Paris (FR)
(72) Inventeur: CANDAU, Didier, F-91570 Bièvres (FR)
(74) Mandataire: Miszputen, Laurent
(86) Numéro de dépôt international: PCT/FR2000/000257
(87) Numéro de publication internationale: WO 2000/047176

(56) Documents cités:
- EP-A- 0 711 804
- EP-A- 0 742 003
- EP-A- 0 775 698
- EP-A- 0 878 469
- WO-A-94/06404
- WO-A-98/22447
- WO-A-98/23252
- WO-A-99/08653

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre, (a) un dérivé de benzotriazole à titre de premier filtre, et (b) un dérivé de bis-résorcinyl triazine particulier à titre de deuxième filtre.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV).

On connaît dans le brevet EP-B-0742003 des compositions cosmétiques antisolaires présentant des facteurs de protection solaire élevés ; celles-ci comprennent, l'association de deux filtres solaires à savoir (a) de l'acide benzène 1,4-di(3-méthylidène-10-camphosulfonique), éventuellement sous forme partiellement ou totalement neutralisée, à titre de premier filtre, et (b), à titre de deuxième filtre, une silicone benzotriazole telle que celles décrites dans le brevet EP-B-0660701. Cependant, ces produits antisolaires ont une rémanence à l'eau insuffisante.

La Demanderesse a découvert, de façon inattendue et surprenante, que la combinaison (a) d'un dérivé de benzotriazole selon la revendication 1 à titre de premier filtre, et (b) d'un dérivé de bis-résorcinyl triazine particulier selon la revendication 1, à titre de deuxième filtre, permettait d'obtenir des compositions antisolaires présentant une rémanence à l'eau sensiblement améliorée tout en ayant un niveau de protection solaire aussi élevé que celui des compositions photoprotectrices de l'art antérieur évoquées ci-dessus.

Cette découverte est à la base de la présente invention.

Ainsi, conformément à l'un des objets de la présente invention, il est maintenant proposé de nouvelles compositions cosmétiques ou dermatologiques, en particulier antisolaires, qui sont essentiellement caractérisées par le fait qu'elles comprennent, dans un support cosmétiquement acceptable:
(a) à titre de premier filtre, au moins un dérivé de benzotriazole de formule (I') selon la revendication 1,
(b) à titre de deuxième filtre, au moins la 2,4-bis-{[4-(2-ethyl-hexyloxy)-2-hydroxy]-phenyl}-6-(4-methoxyphenyl)-1,3,5-triazine.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaîtront à la lecture de la description détaillée qui va suivre.

Le dérivé de benzotriazole de formule (I') conforme à l'invention est un filtre déjà connu en soi. Il est décrit et préparé selon les synthèses indiquées dans les brevets US-4316033 et US-4328346 ; EP-B-0354145 et EP-B-0392883, EP-B-0660701.

Le dérivé de benzotriazole conforme à l'invention a pour formule générale suivante : 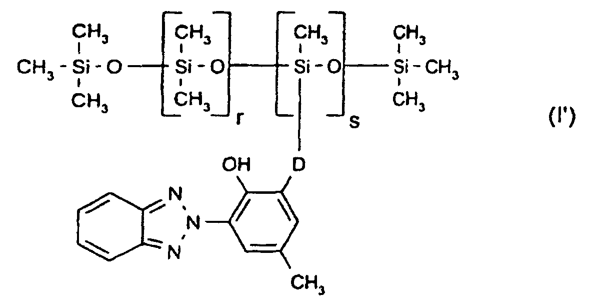 dans laquelle :
r=0
s=1

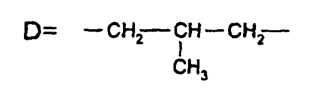

Le dérivé de benzotriazole de formule (I') peut être présent à des teneurs comprises entre 0,1 et 15%, de préférence entre 0,2 et 10%, en poids, toujours par rapport au poids total de la composition.

Le dérivé de bis-résorcinyl triazine de l'invention est un filtre déjà connu en soi. Il est décrit et préparé selon les synthèses indiquées dans la demande de brevet EP-A-0775698.

Le filtre solaire du type dérivés de bis-résorcinyl triazine peut être présent dans les compositions selon l'invention à une concentration comprise entre 0,1 et 15%, de préférence entre 0,2 et 10%, en poids par rapport au poids total de la composition.

Comme indiqué précédemment, selon une caractéristique essentielle de la présente invention, il convient que les deux types de filtres solaires soient tous deux présents dans la composition finale dans une proportion respective telle qu'un effet de synergie au niveau la rémanence à l'eau conférée par l'association résultante, soit obtenu de manière notable, substantielle et significative.

En outre, et d'une manière générale, on notera que les concentrations et rapports en dérivé de benzotriazole de formule (I') et dérivé de bis -résorcinyl triazine tels que définis précédemment sont choisis de manière telle que l'indice de protection solaire de la composition finale soit de préférence d'au moins 2.

Selon un mode préféré de réalisation de la présente invention, le support cosmétiquement acceptable dans lequel se trouvent contenus les différents types de filtres est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques ; les dérivés salicyliques, les dérivés du camphre ; les dérivés de triazine autres que ceux définis précédemment tels que ceux décrits dans les demandes de brevet EP863145, EP517104, EP570838 et EP796851 ; les dérivés de la benzophénone ; les dérivés du dibenzoylméthane ; les dérivés de β,β'-diphénylacrylate, les dérivés de benzimidazole ; les dérivés de l'acide p-aminobenzoïque ; les polymères filtres et silicones filtres tels que ceux décrits dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-boman-2-on-méthylsulfate,
l'acide benzène 1,4-di(3-méthylidène-10-camphosulfonique) et ses sels,
l'acide urocanique,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxoborn-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3-benzylidène-d,l-camphre,
la 2,4,6-tris-[ p-(2'-éthy(hexyl-1'-oxycarbonyl)anilino]-1,3,5-triazine,
2-[p-(tertiobutylamido)anilino]-4,6-bis-[(p-(2'-éthylhexyl-1'-oxycarbonyl)anilino]-1,3,5-triazine,
l'acide 1,4-bis-benzimidazolyl-phènylèn-3,3',5,5'-tétrasulfonique et ses sels,
le polymère de N-[(2 et 4)-[(2-oxobom-3-ylidèn)méthyl] benzyl]-acrylamide.
les polyorganosiloxanes à fonction malonate.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly- -oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier la rémanence à l'eau et le niveau de photoprotection, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de poudre, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

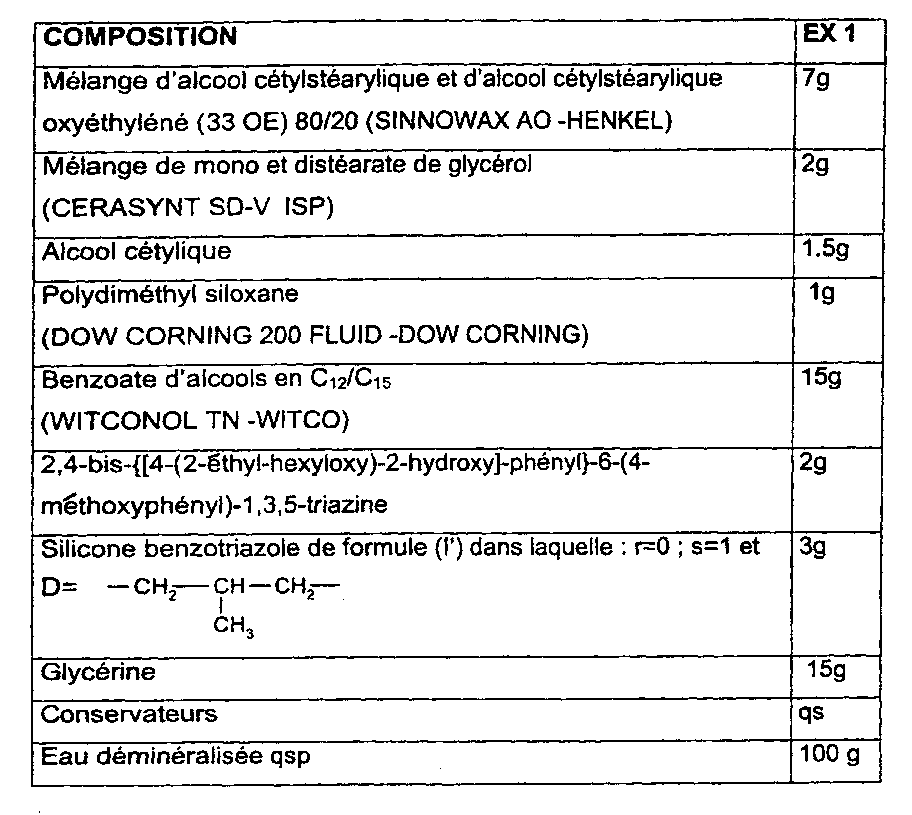

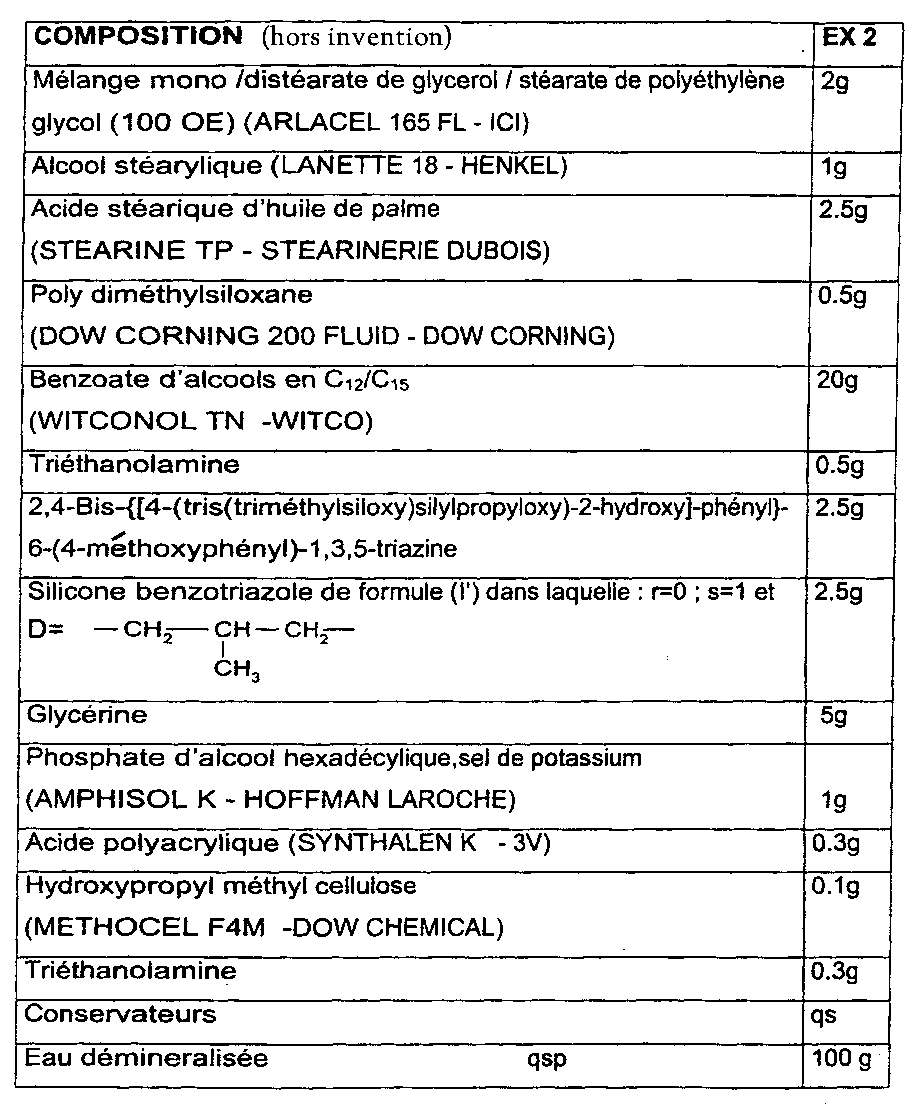

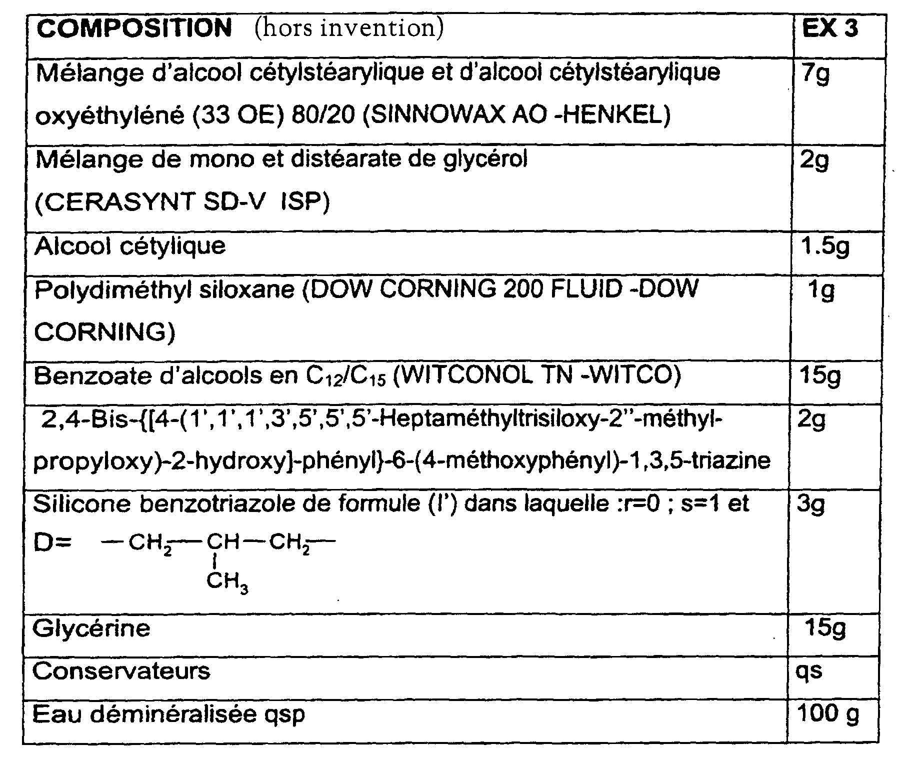

## Revendications

1. Composition cosmétique à usage topique, en particulier pour la photoprotection de la peau et/ou des cheveux, **caractérisée par le fait qu'**elle comprend, dans un support cosmétiquement acceptable:
(a) à titre de premier filtre, **au moins un dérivé de benzotriazole de formule (I') :** 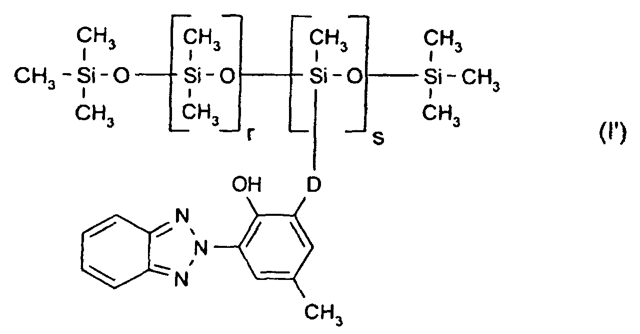
**dans laquelle r=0 ; s=1 et** 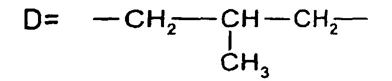 et (b) à titre de deuxième filtre, au moins la 2,4-bis {[4-(2-éthyl-hexyloxy)-2-hydroxy]-phényl}-6-(4-méthoxy-phényl)-1,3,5-triazine.

2. Composition selon la revendication 1, **caractérisée par le fait que** la concentration en dérivé de benzotriazole de formule (I') est comprise entre 0,1 et 15 % en poids par rapport au poids total de la composition.

3. Composition selon la revendication 2, **caractérisée par le fait que** ladite concentration est comprise entre 0,2 et 10 % en poids par rapport au poids total de la composition.

4. Composition selon l'une quelconque des revendications 1 à 3, **caractérisée par le fait que** la concentration en dérivé de bis-résorcinyl triazine est comprise entre 0,1 et 15 % en poids par rapport au poids total de la composition.

5. Composition selon la revendication 4, **caractérisée par le fait que** ladite concentration est comprise entre 0,2 et 10 % en poids par rapport au poids total de la composition.

6. Composition selon l'une quelconque des revendications 1 à 5, **caractérisée par le fait que** ledit support cosmétiquement acceptable se présente sous la forme d'une émulsion de type huile-dans-eau.

7. Composition selon l'une quelconque des revendications 1 à 6, **caractérisée par le fait qu'**elle comprend en outre un ou plusieurs filtres organiques complémentaires actifs dans l'UV-A et/ou UV-B, hydrophiles ou lipophiles, différents desdits premier et deuxième filtres.

8. Composition selon la revendication 7, **caractérisée par le fait que** lesdits filtres organiques complémentaires sont choisis parmi les dérivés cinnamiques, les dérivés salicyliques, les dérivés du camphre, les dérivés de triazine autres que ceux définis dans les revendications précédentes, les dérivés de benzimidazole, les dérivés de la benzophénone, les dérivés du dibenzoylméthane, les dérivés de β,β'-diphénylacrylate, les dérivés de l'acide p-aminobenzoïque, les polymères filtres et silicones filtres.

9. Composition selon l'une quelconque des revendications 1 à 8, **caractérisée par le fait qu'**elle comprend en outre, à titre d'agents photoprotecteurs UV complémentaires, des pigments ou des nanopigments d'oxydes métalliques, enrobés ou non.

10. Composition selon la revendication 9, **caractérisée par le fait que** lesdits pigments ou nanopigments sont choisis parmi les oxydes de titane, de zinc, de fer, de zirconium, de cérium et leurs mélanges, enrobés ou non.

11. Composition selon l'une quelconque des revendications 1 à 10, **caractérisée par le fait qu'**elle comprend en outre au moins un agent de bronzage et/ou de brunissage artificiel de la peau.

12. Composition selon l'une quelconque des revendications 1 à 11, **caractérisée par le fait qu'**elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

13. Composition selon l'une quelconque des revendications 1 à 12, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

14. Composition selon l'une quelconque des revendications 1 à 12, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

15. Composition selon l'une quelconque des revendications 1 à 12, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

16. Utilisation de la composition définie selon l'une quelconque des revendications 1 à 15 pour la fabrication de, compositions cosmétiques pour la protection de la peau et/ou des cheveux contre le rayonnement ultraviolet, en particulier le rayonnement solaire.

17. Procédé de traitement cosmétique pour protéger la peau et/ou les cheveux contre le rayonnement ultraviolet, en particulier le rayonnement solaire, **caractérisé en ce qu'**il consiste à appliquer sur ceux-ci une quantité efficace d'une composition telle que définie à l'une quelconque des revendications 1 à 15.

## Patentansprüche

1. Kosmetische Zusammensetzung zur topischen Verwendung, insbesondere für den Lichtschutz der Haut und/oder der Haare, **dadurch gekennzeichnet, dass** sie in einem kosmetisch akzeptablen Träger enthält:
(a) als erstes Filter mindestens ein Benzotriazolderivat der Formel (I'): 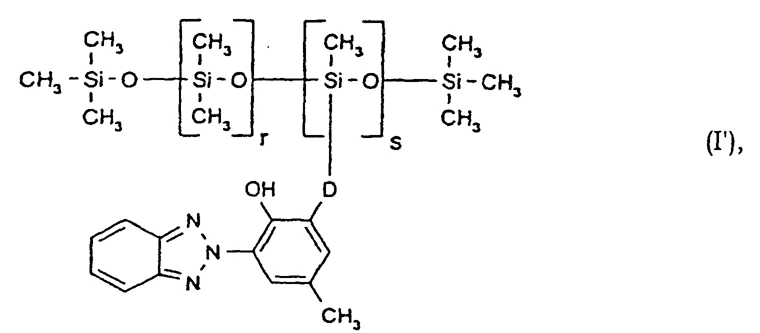 in der bedeuten:
r = 0; s = 1 und 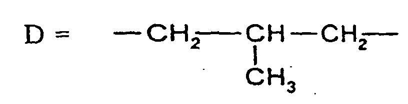
und
(b) als zweites Filter mindestens 2,4-Bis{[4-(2-ethylhexyloxy)-2-hydroxy]-phenyl}-6-(4-methoxyphenyl)-1,3,5-triazin.

2. Zusammensetzung nach Anspruch 1, **dadurch gekennzeichnet, dass** die Konzentration an Benzotriazolderivat der Formel (I') im Bereich von 0,1 bis 15 Gew.-%, bezogen auf das Gesamtgewicht der Zusammensetzung, liegt.

3. Zusammensetzung nach Anspruch 2, **dadurch gekennzeichnet, dass** diese Konzentration im Bereich von 0,2 bis 10 Gew.-%, bezogen auf das Gesamtgewicht der Zusammensetzung, liegt.

4. Zusammensetzung nach einem der Ansprüche 1 bis 3, **dadurch gekennzeichnet, dass** die Konzentration an Bis-resorcinyl-triazin-Derivat im Bereich von 0,1 bis 15 Gew.-%, bezogen auf das Gesamtgewicht der Zusammensetzung, liegt.

5. Zusammensetzung nach Anspruch 4, **dadurch gekennzeichnet, dass** diese Konzentration im Bereich von 0,2 bis 10 Gew.-%, bezogen auf das Gesamtgewicht der Zusammensetzung, liegt.

6. Zusammensetzung nach einem der Ansprüche 1 bis 5, **dadurch gekennzeichnet, dass** der kosmetisch akzeptable Träger in Form einer Emulsion vom Öl-in-Wasser-Typ vorliegt.

7. Zusammensetzung nach einem der Ansprüche 1 bis 6, **dadurch gekennzeichnet, dass** sie außerdem ein oder mehrere ergänzende hydrophile oder lipophile, im UV-A und/oder UV-B-Bereich wirksame organische Filter enthält, die von den oben genannten ersten und zweiten Filtern verschieden sind.

8. Zusammensetzung nach Anspruch 7, **dadurch gekennzeichnet, dass** die ergänzenden organischen Filter ausgewählt sind unter den Zimtsäurederivaten, den Salicylsäurederivaten, den Campherderivaten, den Triazinderivaten, die von den Triazinderivaten verschieden sind, die in den vorhergehenden Ansprüchen definiert sind, den Benzimidazolderivaten, den Benzophenonderivaten, den Dibenzoylmethanderivaten, den β,β'-Diphenylacrylatderivaten, den p-Aminobenzoesäurederivaten, den Polymerfiltern und Siliconfiltern.

9. Zusammensetzung nach einem der Ansprüche 1 bis 8, **dadurch gekennzeichnet, dass** sie außerdem als ergänzende UV-Lichtschutzmittel Pigmente oder Nanopigmente von Metalloxiden, die umhüllt oder nicht umhüllt sind, enthält.

10. Zusammensetzung nach Anspruch 9, **dadurch gekennzeichnet, dass** die Pigmente oder Nanopigmente unter den Oxiden von Titan, Zink, Eisen, Zirconium, Cer und deren Gemischen, die umhüllt oder nicht umhüllt sind, ausgewählt sind.

11. Zusammensetzung nach einem der Ansprüche 1 bis 10, **dadurch gekennzeichnet, dass** sie außerdem mindestens ein Mittel zum Bräunen und/ oder künstlichen Braunfärben der Haut enthält.

12. Zusammensetzung nach einem der Ansprüche 1 bis 11, **dadurch gekennzeichnet, dass** sie außerdem mindestens einen Zusatzstoff enthält, der unter den Fettsubstanzen, den organischen Lösemitteln, den Verdickungsmitteln, den reizlindernden Mitteln, den Antioxidantien, den Trübungsmitteln, den Stabilisierungsmitteln, den Emollientien, den Hydroxysäuren, den Antischaummitteln, den Hydratisierungsmitteln, den Vitaminen, den Parfüms, den Konservierungsmitteln, den grenzflächenaktiven Stoffen, den Füllstoffen, den Maskierungsmitteln, den Polymeren, den Treibmitteln, den alkalisch machenden Mitteln oder den Säuerungsmitteln, den Farbmitteln ausgewählt ist.

13. Zusammensetzung nach einem der Ansprüche 1 bis 12, **dadurch gekennzeichnet, dass** es sich um eine Zusammensetzung zum Schutz der menschlichen Epidermis oder ein Sonnenschutzmittel handelt und dass sie in Form einer nicht-ionischen Vesikeldispersion, einer Emulsion, insbesondere einer Emulsion vom Öl-in-Wasser-Typ, einer Creme, einer Milch, eines Gels, einer Gelcreme, einer Suspension, einer Dispersion, eines Puders, eines festen Stifts, eines Schaumes oder eines Sprays vorliegt.

14. Zusammensetzung nach einem der Ansprüche 1 bis 12, **dadurch gekennzeichnet, dass** es sich um eine Zusammensetzung zum Schminken der Wimpern, der Augenbrauen oder der Haut handelt und dass sie in fester oder pastöser, wasserfreier oder wäßriger Form, in Form einer Emulsion, einer Suspension oder einer Dispersion vorliegt.

15. Zusammensetzung nach einem der Ansprüche 1 bis 12, **dadurch gekennzeichnet, dass** es sich um eine Zusammensetzung handelt, die für den Schutz der Haare vor Ultraviolett-Strahlen vorgesehen ist und dass sie in der Form eines Haarwaschmittels, einer Lotion, eines Gels, einer Emulsion, einer nicht-ionischen Vesikeldispersion vorliegt.

16. Verwendung der Zusammensetzung, die wie in einem der Ansprüche 1 bis 15 definiert ist, für die Herstellung kosmetischer Zusammensetzungen für den Schutz der Haut und/ oder der Haare vor Ultraviolett-Strahlung, insbesondere vor dem Sonnenlicht.

17. Verfahren zur kosmetischen Behandlung für den Schutz der Haut und/oder der Haare vor Ultraviolett-Strahlung, insbesondere Sonnenlicht, **dadurch gekennzeichnet, dass** es darin besteht, auf die Haut und/ oder die Haare eine wirksame Menge einer Zusammensetzung aufzutragen, die wie in einem der Ansprüche 1 bis 15 definiert ist.

## Claims

1. Cosmetic composition for topical use, in particular for the photoprotection of the skin and/or the hair, **characterized in that** it comprises, in a cosmetically acceptable support:
(a) as first screening agent, at least one benzotriazole derivative of formula (I'): 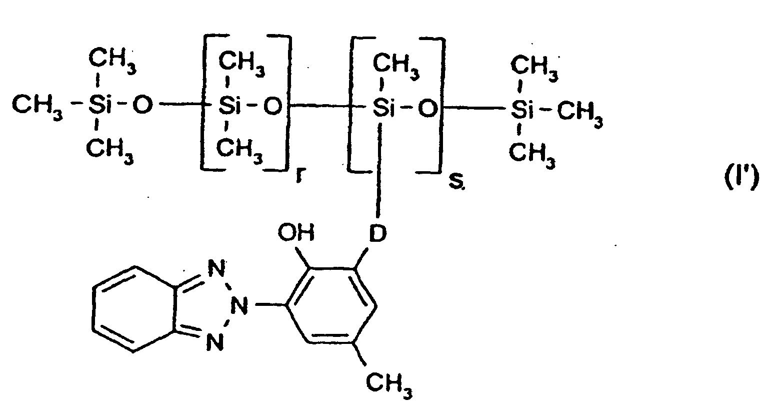
in which r = 0; s = 1 and: 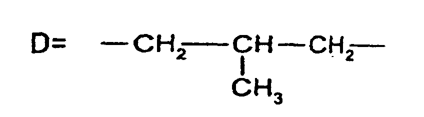 and (b) as second screening agent, at least
- 2,4-bis{[4-(2-ethylhexyloxy)-2-hydroxy]phenyl}-6-(4-methoxyphenyl)-1,3,5-triazine.

2. Composition according to Claim 1, **characterized in that** the concentration of benzotriazole derivative of formula (I') is between 0.1% and 15% by weight relative to the total weight of the composition.

3. Composition according to Claim 2, **characterized in that** said concentration is between 0.2% and 10% by weight relative to the total weight of the composition.

4. Composition according to any one of Claims 1 to 3, **characterized in that** the concentration of bis-resorcinyl triazine derivative is between 0.1% and 15% by weight relative to the total weight of the composition.

5. Composition according to Claim 4, **characterized in that** said concentration is between 0.2% and 10% by weight relative to the total weight of the composition.

6. Composition according to any one of Claims 1 to 5, **characterized in that** said cosmetically acceptable support is in the form of an emulsion of oil-in-water type.

7. Composition according to any one of Claims 1 to 6, **characterized in that** it also comprises one or more additional hydrophilic or lipophilic UV-A-active and/or UV-B-active organic screening agents other than said first and second screening agents.

8. Composition according to Claim 7, **characterized in that** said additional organic screening agents are chosen from cinnamic derivatives, salicylic derivatives, camphor derivatives, triazine derivatives other than those defined in the preceding claims, benzimidazole derivatives, benzophenone derivatives, dibenzoylmethane derivatives, β,β'-diphenylacrylate derivatives, p-aminobenzoic acid derivatives, screening polymers and screening silicones.

9. Composition according to any one of Claims 1 to 8, **characterized in that** it also comprises, as additional UV photoprotective agents, coated or uncoated metal oxide pigments or nanopigments.

10. Composition according to Claim 9, **characterized in that** said pigments or nanopigments are chosen from titanium oxide, zinc oxide; iron oxide, zirconium oxide and cerium oxide, which are coated or uncoated, and mixtures thereof.

11. Composition according to any one of Claims 1 to 10, **characterized in that** it also comprises at least one agent for artificially tanning and/or browning the skin.

12. Composition according to any one of Claims 1 to 11, **characterized in that** it also comprises at least one adjuvant chosen from fatty substances, organic solvents, thickeners, softeners, antioxidants, opacifying agents, stabilizers, emollients, hydroxy acids, anti-foaming agents, moisturizers, vitamins, fragrances, preserving agents, surfactants, fillers, sequestering agents, polymers, propellants, basifying or acidifying agents and dyes.

13. Composition according to any one of Claims 1 to 12, **characterized in that** it is a composition for protecting the human epidermis or an antisun composition and **in that** is in the form of a nonionic vesicular dispersion, an emulsion, in particular an emulsion of oil-in-water type, a cream, a milk, a gel, a cream-gel, a suspension, a dispersion, a powder, a solid stick, a foam or a spray.

14. Composition according to any one of Claims 1 to 12, **characterized in that** it is a make-up composition for the eyelashes, the eyebrows or the skin and **in that** it is in solid or pasty, anhydrous or aqueous form or in the form of an emulsion, a suspension or a dispersion.

15. Composition according to any one of Claims 1 to 12, **characterized in that** it is a composition intended for protecting the hair against ultraviolet rays and **in that** it is in the form of a shampoo, a lotion, a gel, an emulsion or a nonionic vesicular dispersion.

16. Use of the composition defined according to any one of Claims 1 to 15, for the manufacture of cosmetic compositions for protecting the skin and/or the hair against ultraviolet radiation, in particular solar radiation.

17. Cosmetic treatment process for protecting the skin and/or the hair against ultraviolet radiation, in particular solar radiation, **characterized in that** it consists in applying an effective amount of a composition as defined in any one of Claims 1 to 15 to the skin and/or the hair.
